# EUROPEAN PATENT APPLICATION

(11) **EP 0 914 815 A1**
(43) Date of publication of application: **12.05.1999**
(21) Application number: 98911080.4
(22) Date of filing: 30.03.1998
(51) Int. Cl.: A61K 7/00, A61K 7/48, A61K 31/195, A61K 31/235, A61K 31/375

(54) **COMPOSITION FOR EXTERNAL USE FOR PREVENTION OF ENVIRONMENTAL STRESS**

(30) Priority: 30.03.1997 JP 95307/97
(71) Applicant: SHISEIDO COMPANY LIMITED, Chuo-ku, Tokyo 104-8010 (JP)
(72) Inventor: EGAWA, Mariko, Shiseido Research Center (1),, Yokohama-shi Kanagawa 223-8553 (JP); SAKAMOTO, Tetsuo, Shiseido Research Center (1),, Yokohama-shi Kanagawa 223-8553 (JP); KOHNO, Yoshiyuki, Shiseido Research Center (1),, Yokohama-shi Kanagawa 223-8553 (JP)
(74) Representative: TER MEER STEINMEISTER & PARTNER GbR
(86) International application number: JP9801420
(87) International publication number: WO9843597

(57) **Abstract**

A composition for improving environmental stress which contains one or more types of ingredients chosen from among a group consisting of sulfur containing amino acids, metabolic intermediates of sulfur containing amino acids, tannin and vitamin C and its derivatives. Also, said composition for improving environmental stress wherein said sulfur containing amino acid is glutathione and the metabolic intermediate of the sulfur containing amino acid is thiotaurine or hypotaurine. Also, said composition for improving environmental stress wherein hydroxycarboxylic acid or its derivative is additionally added to this composition. The liniment composition for preventing environmental stress pertaining to the present invention is suitable for eliminating the stress which has adverse effects on the skin among types of stress due to floating fine particles which float in the air.

## Description

### FIELD OF THE INVENTION

The present invention belongs in general to the technical field relating to liniment compositions, and more particularly to a liniment composition which can eliminate various adverse effects incurred on the skin by external environmental stress due to floating fine particles, particularly floating fine particles which are generated artificially and float in the air.

### BACKGROUND OF THE INVENTION

In modern society, we have quite a few occasions to be exposed to floating fine particles which are generated artificially and float in the air.

For example, in a closed space, such as an office, where people have many occasions to be exposed to each other in a crowded situation, there are many opportunities to be exposed to floating fine particles from tobacco smoke generated by smoking. That is, if there is only one heavy smoker in an environment crowded with people, such as an office, these people end up being frequently exposed to tobacco smoke directly or indirectly unless there are some active countermeasures such as creating Menphis areas or installing air cleaners.

In cities and in the vicinity of major arterial roads, there are many occasions to be exposed to floating fine particles and nitrogen oxides (NOₓ) from the automobile exhaust gas. Also, these nitrogen oxides and such produce ozone, which in turn becomes so-called night smog, causing problems. Furthermore, in the vicinity of chemical plants, thermal power plants, discontinued mines, industrial waste areas, etc., there are many occasions to be exposed to floating fine particles of soot and smoke as well as heavy metal fine particles.

In an environment where people are exposed to each floating fine particles, contact between these floating fine particles and the skin causes various types of stress on the people in the environment.

For example, Tobacco smoke is known to have various adverse effects on humans such as a cause of carcinogenesis, emphysema, etc., and also it is known to deteriorate the condition of the skin such as by causing wrinkles.

Although it is true to some degree that people maintain adequate alertness by stress, the above described environmental stress due to external causes are nothing but harmful. Therefore, means to eliminate this type of environmental stress are sought after.

While the environmental stress due to these floating fine particles which have adverse effects on the condition of the skin always exists as people go on with their lives, it exists more conspicuously in the aforementioned offices and in the vicinity of factories and arterial roads (hereafter, these may be referred to as "stress environments").

Just as there is more ultraviolet light on summer beaches and winter mountains and "a means to prevent sunburning" is necessary in such an environment, leading to creation of "a sunscreen composition" as a countermeasure, "a means to protect the skin from environmental stress" has to be specifically created in modern society where such stress environments actually exist.

The object of the present invention is to provide a means to efficiently prevent the stress due to floating fine particles which float in the air which has adverse effects on the skin in particular (referred to also as "environmental stress" in the present invention) and to maintain healthy skin even in the stressful environment.

This environmental stress is, for example, a stress which is speculated be caused by various chemical and/or physical factors such as oxidation reactions triggered by the contact between floating fine particles and the skin (there may also be an influence due to psychological factors such as psychological stress from discomfort due to the mere contact with floating fine particles). In any case it is believed that this environmental stress does not, for example, have a single cause such as in the case of photo aging of the skin due to long-term exposure to ultraviolet light from the sun, and that it is a stress deeply related to skin conditions caused by intricately intertwined multiple factors.

The inventors conducted earnest investigation to achieve this object and discovered that certain antioxidants have the effect of efficiently eliminating the adverse effects of the aforementioned environmental stress on the skin and that the skin can be effectively protected from environmental stress by providing a liniment composition which contains these antioxidants (such a liniment composition will be referred to as "a liniment composition for preventing environmental stress"), thus completing the present invention.

### DISCLOSURE OF THE INVENTION

The present invention provides a liniment composition for preventing environmental stress (hereafter referred to as "a liniment composition for preventing environmental stress of the present invention") which contains one or more types of ingredients chosen from among a group consisting of sulfur containing amino acids, metabolic intermediates of sulfur containing amino acids, tannin and vitamin C and its derivatives.

In the liniment composition for preventing environmental stress of the present invention, it is preferable that the aforementioned sulfur containing amino acid be glutathione and the metabolic intermediate of the sulfur containing amino acid be thiotaurine or hypotaurine.

In the liniment composition for preventing environmental stress of the present invention, it is possible to additionally blend hydroxycarboxylic acid or its derivative in the composition.

As mentioned above, in the present invention, "environmental stress" refers to external physical and/or chemical stress factors accompanying human civilization such as the aforementioned tobacco smoke in offices or soot and smoke from factories, rather than internal mental stress accompanying increasingly complex human relationships. The liniment composition for preventing environmental stress of the present invention is a liniment composition to protect the skin from adverse effects due to the aforementioned environmental stress in a stressful environment in order to maintain healthy skin.

The "floating fine particles" which cause "environmental stress" are those which are mainly generated by artificial causes such as tobacco smoke, automobile exhaust (not only from gasoline engines but also from diesel engines) as well as soot and smoke from chemical plants and thermal power plants (also including those due to natural causes such as soot and smoke due to volcano eruptions) and, in general, fine particles which float in the atmosphere (outdoor and indoor) for at least a certain amount of time and contact human skin and have some effect on it. Therefore, a gas which does exist in the atmosphere at normal temperatures but has no effect on human skin upon contact, such as carbon dioxide, is excluded from the definition of "floating fine particles". However, molecules of pollutants such as NOₓ and Oₐ which float in the atmosphere and can have some effect on human skin upon contact are included in the definition of "floating fine particles".

### BRIEF EXPLANATION OF THE DRAWING

FIG. 1 is a schematic drawing of the tobacco smoke exposure apparatus.
FIG. 2 is a figure illustrating the effect of various essential ingredients on suppressing the reduction in the corneum moisture content.
FIG. 3 is a figure illustrating the effect of various essential ingredients on suppressing the ultraweak chemiluminescence of the skin.

### THE BEST MODES OF THE EMBODIMENTS

Embodiments of the present invention are described below.

The liniment composition for preventing environmental stress of the present invention is a composition which contains as essential ingredients thiotaurine, hypotaurine, tannin and/or vitamin C or its derivatives. Examples of the sulfur containing amino acid which can be blended into the liniment composition for preventing environmental stress of the present invention include methionine, cystine, cysteine and glutathione.

It is preferable to choose glutathione among these sulfur containing amino acids for blending into the liniment composition for preventing environmental stress of the present invention.

Examples of the metabolic intermediate of the sulfur containing amino acid which can be blended into the liniment composition for preventing environmental stress of the present invention include homocysteine, sulfinic acid, cysteinic acid, thiocysteine, taurine, djenkolic acid, cystathionine, S-allylcysteine, lanthionine and enthionine.

Examples of the metabolic intermediate of the sulfur containing amino acid also include various aminoethyl compounds. It is preferable that the liniment composition for preventing environmental stress of the present invention contain an aminoethyl compound represented by
NH₂CH₂CH₂X
wherein X denotes a thiosulfonic acid group -SO₂SH, in which case the compound is thiotaurine, or wherein X denotes a sulfinic acid group -SO₂H, in which case the compound is hypotaurine.

Both thiotaurine and hypotaurine are contained in major organs such as the heart, brain and liver of many mammals including humans, and also amply contained in food we eat daily. There have been no reports of side effects such as irritation, itchness or rashes and therefore they are highly safe aminoethyl compounds.

Both thiotaurine and hypotaurine can be easily prepared with a conventional method from thio compounds which are easy to obtain, such as cysteine. It is also possible to blend those commercially available into the liniment composition for preventing environmental stress of the present invention.

Tannin which can be blended into the liniment composition for preventing environmental stress of the present invention is also called tannic acid and these are generic names of complex aromatic compounds with many phenolic hydroxyl groups. Tannin is usually classified into the condensation type tannin which is derived from the polymerization of flavanol skeletons and the hydrolysis type tannin which is derived from the depside bonding of aromatic organic acid and sugar. In the present invention, both of these types of tannin can be blended in.

The source of tannin to be used in the liniment composition for preventing environmental stress of the present invention is not limited either. Possible sources include tea, gallnut and persimmon, but the present invention is not limited to these.

Among tannins from various sources, tannin derived from tea is a particularly preferable tannin to be blended into the liniment composition for preventing environmental stress of the present invention.

The aforementioned tannin to be blended into the liniment composition for preventing environmental stress of the present invention can either be extracted and prepared using a conventional prior art method or commercially obtained.

The vitamin C and derivatives which can be blended into the liniment composition for preventing environmental stress of the present invention include L-ascorbic acid, ascorbates such as sodium ascorbate, ascorbic acid derivatives including L-ascorbic acid alkyl ester, L-ascorbic acid phosphoric ester, L-ascorbic acid sulfuric ester and L-ascorbic acid glucoside.

Specific examples of the aforementioned L-ascorbic acid alkyl ester include L-ascorbyl palmitate, L-ascorbyl isopalmitate, L-ascorbyl dipalmitate, L-ascorbyl diisopalmitate, L-ascorbyl stearate, L-ascorbyl isostearate, L-ascorbyl distearate, L-ascorbyl myristate, L-ascorbyl isomyristate, L-ascorbyl dimyristate, L-ascorbyl diisomyristate, L-ascorbyl 2-ethylhexanoate, L-ascorbyl di-2-ethylhexanoate, L-ascorbyl oleate and L-ascorbyl dioleate.

Specific examples of the aforementioned L-ascorbic acid phosphoric ester include L-ascorbic acid 2-phosphoric ester, L-ascorbic acid 3-phosphoric ester and DL-α-tocopherol-2-L-ascorbic acid diphosphoric ester.

Specific examples of the aforementioned L-ascorbic acid sulfuric ester include L-ascorbic acid 2-sulfuric ester and L-ascorbic acid 3-sulfuric ester.

In the liniment composition for preventing environmental stress of the present invention, it is also possible to blend in salts of these derivatives of ascorbic acid. Examples include alkali metal salts and alkali earth metal salts, such as sodium salts, potassium salts, calcium salts and magnesium salts, of the aforementioned ascorbic acid derivatives.

The aforementioned vitamin C and derivatives to be blended into the liniment composition for preventing environmental stress of the present invention can be either extracted and/or manufactured according to prior art methods, or purchased commercially.

As described thus far, the liniment composition for preventing environmental stress of the present invention contains as essential ingredients sulfur containing amino acids, metabolic intermediates of sulfur containing amino acids, tannin or vitamin C or its derivatives. These ingredients can either be blended in singly or in combinations of two or more.

As for the content of the essential ingredients in the aforementioned liniment composition for preventing environmental stress of the present invention, the content of the sulfur containing amino acid or the metabolic intermediate of the sulfur containing amino acid is 0.001 wt% or more and 5.0 wt% or less of the total liniment composition.

The content of tannin is 0.0001 wt% or more and 5.0 wt% or less of the total liniment composition.

The content of vitamin C or its derivatives is 0.001 wt% or more and preferably 10.0 wt% or less of the total liniment composition.

When these ingredients are blended in combination, the content of each ingredient can be selected according to the specific combination.

As described thus far, the liniment composition for preventing environmental stress of the present invention containing the aforementioned ingredients can effectively eliminate the adverse effects incurred on human skin in a stress environment. The protection against the adverse effects in the stress environment can be further improved by addition of hydroxycarboxylic acid and/or its derivatives to the aforementioned liniment composition for preventing environmental stress of the present invention.

Examples of the aforementioned hydroxycarboxylic acid and/or its derivatives to be blended in the liniment composition for preventing environmental stress of the present invention include hydroxycarboxylic acid or its ester, lactone, salts, etc.

Specific examples include glycolic acid, benzilic acid, tropic acid, lactic acid, malic acid, citric acid, isocitric acid, citramalic acid, tartronic acid, tartaric acid, gluconic acid, galactonic acid, α -hydroxyiso butylic acid, phenyl-lactic acid, muldic acid, atrolactic acid, gluconolactone, galactonolactone, ribonic acid, ribonolactone, pantoic acid, pantolactone, pantotheinic acid, α -hydroxybutylic acid, β -hydroxybutylic acid, quinic acid and pyruvic acid, phenylpyruvic acid, methyl pyruvate, ethyl pyruvate, benzoylformic acid, methyl benzoylformate and ethyl benzoylformate.

Among these hydroxycarboxylic acid and its derivatives, more preferable in the present invention are glycolic acid, lactic acid, malic acid and citric acid.

The aforementioned hydroxycarboxylic acid and its derivatives can be either singly blended in the liniment composition for preventing environmental stress of the present invention or in combination of two or more as necessary. The content of the aforementioned hydroxycarboxylic acid and its derivatives in the liniment composition for preventing environmental stress of the present invention is not limited in particular. It should be selected based on the content of the aforementioned sulfur containing amino acid and the combination. In general, it should be 0.001 wt% or more and 1.0 wt% or less of the total liniment composition.

In order to obtain the expected effect of the present invention, i.e. elimination of the adverse effects in a stress environment, it is not necessary to blend in any other ingredients in the liniment composition for preventing environmental stress of the present invention. However, other ingredients can be blended in for the purpose of giving the effect they generally have to the liniment composition for preventing environmental stress of the present invention, as long as the expected effect of the present invention is not affected.

In this sense, ultraviolet light absorbents, ultraviolet light blocking agents, humectants, vitamins other than vitamin C, anti-inflammatory agents, plant extracts other than tannin and skin activating agents can be blended into the liniment composition for preventing environmental stress of the present invention.

Also, the liniment composition for preventing environmental stress of the present invention can take every form of endermic liniment (cosmetics, drugs, quasi-drugs). Specifically, it can be used in a lotion, emulsion, cream, ointment, pack, aerozol, water-oil two-phase agent, water-oil-powder three-phase agent, essence, gel, foundation, lip stick, shampoo, rinse, etc.

According to these specific forms, various general base agents and such can be blended into the liniment composition for preventing environmental stress of the present invention within the range which does not affect the expected effect of the present invention. Specific examples include liquid oil/fat, solid oil/fat, waxes, ester oil, hydrocarbon oil, silicone oil, silicone, anioninc surfactants, cationic surfactants, ampholytic surfactants, non-ionic surfactants, lower alcohols, sterols, water soluble polymers, sequestering agents, neutralizing agents, pH adjusting agents, antimicrobial agents, perfumes and pigments.

These base agents and such are blended into the liniment composition for preventing environmental stress of the present invention in combination according to the recipe designed for the desired form.

Specific recipes of the liniment composition for preventing environmental stress of the present invention are described in the following examples.

### EXAMPLES

Specific descriptions of the present invention are given below by referring to examples and such. The technological range of the present invention should not be interpreted as limited to those examples.

### [Test example]

### A. Testing of the influence that floating fine particles have on conditions of the skin

The following experiment was conducted to capture, as a phenomenon on the skin, the influence of floating fine particles on conditions of the skin in a stressful environment where the skin is exposed to tobacco smoke and/or exhaust gas at high frequencies.

### A-1. Testing of the influence on the corneum moisture content

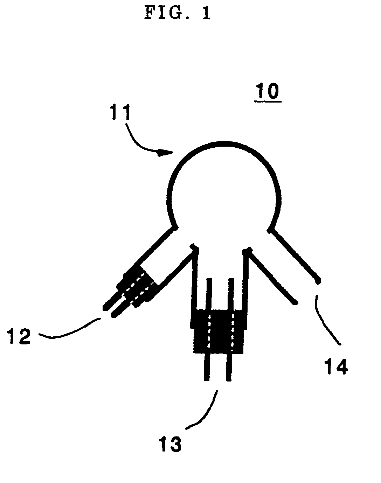

The floating fine particles exposure apparatus 10 shown in FIG. 1 was used to expose a testee's skin to tobacco (with 24 mg of tar and 2.4 mg of nicotine) smoke or exhaust gas fine particles sampled from an exhaust outlet of an automobile for 10 minutes per day for three months consecutively by bringing the medial aspect of the testee's forearm near the discharge port 14 of the floating fine particles of this exposure apparatus 10 (Exposure apparatus 10 uses a three-mouthed flask 11. That is, one of the mouths of the three-mouthed flask is used as an air inlet 12, another is used as an inlet 13 for floating fine particles such as tobacco smoke, and the third mouth is used as a discharge port 14. Floating fine particles such as tobacco smoke flow in through inlet 13 of the floating fine particles and an air flow which flows in through air inlet 12 is used to discharge them through open discharge port 14 of the floating fine particles. An object which is brought close to this discharge port 14 can be exposed to the floating fine particles such as tobacco smoke.

Changes in the corneum moisture content after consecutive exposure to tobacco smoke or exhaust gas fine particles for the aforementioned three months were analyzed by using a Corneometer™ (Courage and Khazaka, Germany).

The corneum moisture content is one of the indicators of skin conditions and is known to be an indicator which allows determination of the amount of moisture in the corneum. That is, in the case of rough skin, the corneum moisture content is smaller. A higher value of the corneum moisture content indicates better skin conditions.

The aforementioned testing indicated that an approximately 20% or more reduction in the corneum moisture content was observed in the group which was exposed to tobacco smoke and an approximately 10% or more reduction in the corneum moisture content was observed in the group which was exposed to exhaust gas. It became clear that both exposed groups clearly suffered a reduction in the corneum moisture content and therefore an aggravation of their skin conditions.

Also, aggravation of the appearance of the skin, such as scaling, was observed in the groups which were exposed to tobacco smoke or exhaust gas.

This testing made it clear that, in a stressful environment, a relatively long-term contact of the skin with floating fine particles causes adverse effects on conditions of the skin. The aforementioned testing made it clear that, when a "stressful environment" occurs, (1) the skin is exposed to the stressful environment where it frequently comes into contact with floating fine particles, and that (2) aggravation of appearance such as rough skin, lackluster skin and reddening and/or some adverse effects such as a reduction in the corneum moisture content are observed.

### A-2. Testing of the oxidation reactions on the skin

In this testing, oxidation reactions on the skin in a stressful environment were investigated.

Based on an epidemiologic report to the effect that smokers, compared with nonsmokers, have more wrinkles and poor conditions of the skin (Donald P. Kaudunce, MD et. al., ; Annals of Internal Medicine; 114: 840-844 (1991) and a report to the effect that many radicals are contained in tobacco smoke (William A. Pryor et. al.,; Environmental Health Perspectives:64:111-126 (1985)), testing was conducted to determine whether or not oxidation reactions due to radicals are observed on the skin in an environment in which people are very frequently exposed to tobacco smoke, such as an office with a heavy smoker in it.

It is known that a phenomenon called "ultraweak chemiluminescence: CL" occurs on the skin of a living body including a human. This ultraweak chemiluminescence is known to occur in the radiation deactivation process of electrons from the excited state to the ground state.

For example, radicals such as singlet oxygen, excited carbonic acid, excited carbonyl, etc. are known to be this luminescent substance in a living body. Therefore, if this ultraweak chemiluminescence can be monitored, it is possible to adequately identify the oxidation reactions occurring on the human skin.

Based on this principle, in this testing, the degree of the aforementioned ultraweak chemiluminescence due to tobacco smoke in human fibroblast cells, which perform an important role in maintaining human skin, was determined and thus the oxidation reactions on the skin under a stressful environment were investigated.

Specifically, human fibroblast cells (NB1RGB) were cultured in a MEM medium in a 35 mm Petri dish until they were subconfluent. After this culture, the medium for them was replaced by a new MEM medium with the same composition and then an overnight culture was carried out under the same conditions as above.

After the culture was completed, the medium was removed and replaced with PBS (-) and the ultraweak chemiluminescence from the aforementioned human fibroblast cells adhered to the bottom of the Petri dish was measured for 10 minutes using a chemiluminescence detector (model CLD-110 from Tohoku Electronic Industries Co., Ltd.). The number of photoelectrons generated during this time was converted to an electric voltage to obtain the blank value of the ultraweak chemiluminescence.

Using the exposure apparatus for floating fine particles shown in FIG. 1, the human fibroblast cells in the Petri dish(s) were then exposed to tobacco (with 24 mg of tar and 2.4 mg of nicotine) smoke from discharge port 14 for 90 seconds. Thirty seconds after the exposure, the ultraweak chemiluminescence from the cells was measured for 30 minutes in the same manner using the chemiluminescence detector.

As a result, hardly any ultraweak chemiluminescence was observed in the control group which had not been exposed to tobacco smoke. However, more than normal ultraweak chemiluminescence was observed in the group which had been exposed. This fact was discovered by the inventors for the first time.

The exposure apparatus for floating fine particles shown in FIG. 1 which was used in the aforementioned testing was used to conduct testing identical to the aforementioned testing except for the fact that exhaust gas fine particles sampled from an exhaust outlet of an automobile engine were used instead of tobacco. As was the case with the aforementioned tobacco smoke, more than normal ultraweak chemiluminescence was observed in the human fibroblast cells, indicating excessive oxidation reactions.

### B. Testing of environmental stress suppression

### B-1. Testing of suppression of a reduction in the corneum moisture content

An 1.0 wt% aqueous solution of each of 1) α - tocopherol, 2) β -carotene, 3) thiotaurine (from Sogo Pharmaceutical Co., Ltd.), 4) hypotaurine (from Sogo Pharmaceutical Co., Ltd.), 5) glutathione, 6) tannin, 7) vitamin C derivative [L-ascorbic acid phosphate (magnesium)], 8) thiotaurine + malic acid, 9) hypotaurine + malic acid and 10) glutathione + malic acid (when combined with malic acid, 1.0 wt% of malic acid was contained as well as 1.0 wt% the antioxidant) was applied on the medial aspect of a human forearm. For each of these, the blank value of the corneum moisture content was measured. For the controls, "the group with no drug applied" and "the non-exposure group", which was not exposed to tobacco smoke or exhaust gas, were used.

Using the tobacco smoke exposure apparatus shown in the aforementioned FIG. 1, the medial aspect of the human forearm onto which the aforementioned ingredients had been applied was exposed to tobacco smoke or exhaust gas for 10 minutes a day for three months and the corneum moisture content of that area was investigated.

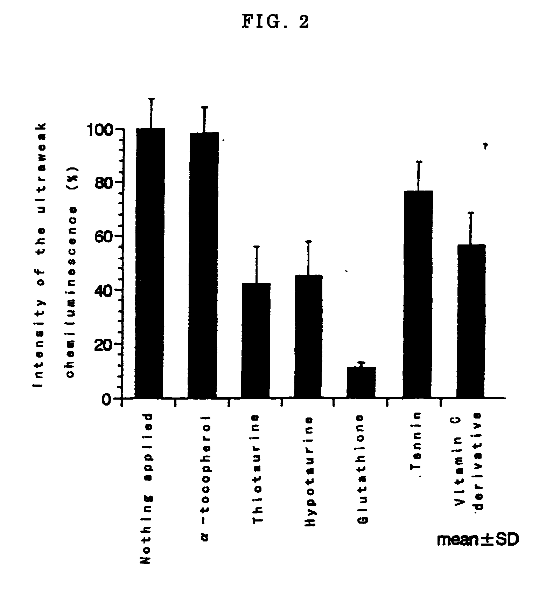

The results are shown in FIG. 2.

FIG. 2 indicates that the aggravation of skin conditions which would occur due to exposure to tobacco smoke or exhaust gas was effectively Prevented by certain antioxidants, specifically 3) thiotaurine (from Sogo Pharmaceutical Co., Ltd.), 4) hypotaurine (from Sogo Pharmaceutical Co., Ltd.), 5) glutathione, 6) tannin and 7) vitamin C derivative [L-ascorbic acid phosphate (magnesium)).

Although 1) α -tocopherol and 2) β -carotene are known to be substances with very strong antioxidant actions, it was clearly shown that these could only minimally suppress the aggravation of skin conditions due to exposure to tobacco smoke or exhaust gas.

It has become clear from these results that this suppression affect on the reduction in the corneum moisture content was improved by blending in hydroxycarboxylic acid in addition to these antioxidants.

Thus, it became clear that certain antioxidants were very effective to suppress environmental stress.

### B-2. Testing of suppression of oxidation

An 1.0 wt% aqueous solution of each of 1) thiotaurine (from Sogo Pharmaceutical Co., Ltd.), 2) hypotaurine (from Sogo Pharmaceutical Co., Ltd.), 3) glutathione, 4) tannin and 5) vitamin C derivative [L-ascorbic acid phosphate (magnesium)] was applied on the medial aspect of a human forearm. For each of these, a chemiluminescence detector (model OX-7 from Tohoku Electronic Industries Co., Ltd.) whose sample chamber had been modified for a human arm was used to carry out a 30 minute measurement to obtain the blank value of the ultraweak chemiluminescence for each of them. For the control, a solution of 1.0 wt% α -tocopherol was used.

In the same manner as in the aforementioned environmental stress suppression testing which used the corneum moisture content of the skin as the indicator, the medial aspect of a human forearm was brought close to the floating fine particles exposure apparatus shown in FIG. 1 to expose the medial aspect of the forearm, onto which the aforementioned ingredient(s) had been applied, to tobacco smoke for 90 seconds. Sixty seconds after exposure, the ultraweak chemiluminescence of the exposed part was measured for 30 minutes in the same manner as described above.

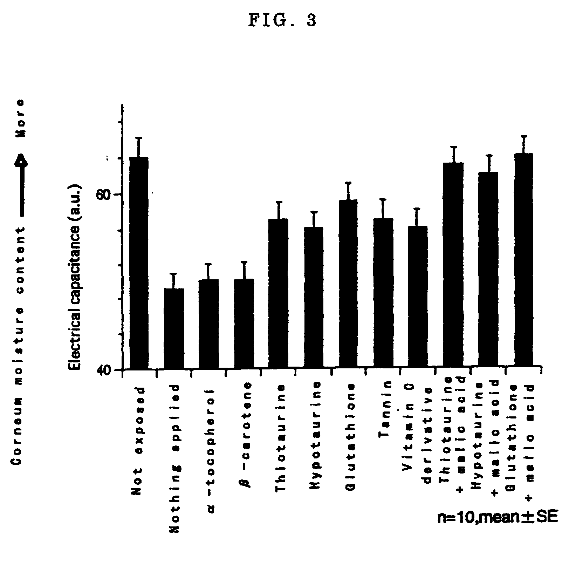

The results are shown in FIG. 3. In FIG. 3, the vertical axis represents the intensity of the ultraweak chemiluminescence [a relative value (%) assuming the blank value without anything applied to be 100] and along the horizontal axis are the essential ingredients of the liniment composition for preventing environmental stress of the present invention for which the testing was conducted. Among these essential ingredients, the vitamin C derivative refers to the aforementioned L-ascorbic acid phosphate (magnesium).

It has become clear from these results that the essential ingredient(s) in each liniment composition for preventing environmental stress of the present invention significantly reduces the ultraweak chemiluminescence on the skin. That is, it has become clear that each of the aforementioned ingredients significantly reduces the oxidation reactions on the skin.

The reduction of the ultraweak chemiluminescence is particularly significant with glutathione and thiotaurine, indicating they are useful.

As described thus far, it has become clear that certain antioxidants can suppress the oxidation reactions on the skin which occur due to coming to contact with floating fine particles in a stressful environment and will result in adverse effects such as skin aging.

However, the results of this oxidation suppression testing and the results of the aforementioned investigation on the corneum moisture content are not necessarily in agreement. For example, tannin and vitamin C derivatives, which didn't exhibit very distinctive antioxidation effects, produced good results in the testing of the corneum moisture content, which directly indicates the degree of suppression of actual adverse effects due to the environmental stress.

Also, α -tocopherol itself, which was used as a control for this oxidation suppression testing, is known to be an excellent antioxidant, equivalent or better compared with the other antioxidants used for test samples in the testing. However, it could not effectively suppress the oxidation reactions on the skin.

Recipe examples for the liniment composition for preventing environmental stress of the present invention are described below. The same testing as in the aforementioned testing was conducted for these liniment compositions for preventing environmental stress of the present invention and significant suppression of the aggravation of skin conditions (an increase in the corneum moisture content) was observed for all the liniment compositions.

### [Example 1] Lotion

| (Ingredients) | Blended amount (wt% of the total liniment composition. The same applies for the rest as well.) |
|---|---|
| (1) Tocopherol acetate | 0.01 |
| (2) Glycerine | 4.0 |
| (3) 1,3-butylene glycol | 4.0 |
| (4) Thiotaurine | 0.1 |
| (5) Ethanol | 7.0 |
| (6) Polyoxyethylene (18) oleyl alcohol ether | 0.5 |
| (7) Methyl paraben | 0.2 |
| (8) Citric acid | 0.05 |
| (9) Sodium citrate | 0.1 |
| (10) Perfume | 0.05 |
| (11) Purified water | Balance |

### 〈Preparation method〉

Citric acid, sodium citrate, glycerine, and 1,3-butylene glycol were dissolved in purified water (water phase). Separately, ethanol, polyoxyethylene (18) oleyl alcohol ether, tocopherol acetate, perfume and methyl paraben were dissolved and added to said water phase, followed by solubilization and filtering to obtain the aforementioned lotion.

### [Example 2] Cream

| (Ingredients) | Blended amount (wt%) |
|---|---|
| (1) Cetostearyl alcohol | 3.5 |
| (2) Squalane | 40.0 |
| (3) Bees wax | 3.0 |
| (4) Reduced lanolin | 5.0 |
| (5) Ethyl paraben | 0.3 |
| (6) Polyoxyethylene (20) sorbitan monopalmitate | 2.0 |
| (7) Monoglyceride stearate | 2.0 |
| (8) Sodium N-stearoylglutamate | 0.5 |
| (9) 2-hydroxy-4-methoxybenzophenone | 1.0 |
| (10) Thiotaurine | 0.1 |
| (11) Perfume | 0.05 |
| (12) 1,3-butylene glycol | 5.0 |
| (13) Purified water | Balance |

### 〈Preparation method〉

(1)-(10) were dissolved by heating and, while being stirred, added to (11)-(13) heated up to 70°C. This was treated with a homogenizer to make the emulsion particles finer, followed by rapid cooling with stirring to obtain the aforementioned cream.

### [Example 3] Emulsion

| (Ingredients) | Blended amount (wt%) |
|---|---|
| (1) Stearic acid | 1.5 |
| (2) Cetyl alcohol | 0.5 |
| (3) Bees wax | 2.0 |
| (4) Polyoxyethylene (10) monooleate | 1.0 |
| (5) Octyl methoxycinnamate | 2.0 |
| (6) Thiotaurine | 0.1 |
| (7) Triethanolamine | 0.75 |
| (8) Glycerine | 7.0 |
| (9) Ethyl paraben | 0.3 |
| (10) Perfume | 0.03 |
| (11) Purified water | Balance |

### 〈Preparation method〉

Thiotaurine, glycerine and triethanolamine were added to purified water and the temperature was maintained at 70°C(water phase). Other ingredients were mixed and dissolved by heating and the temperature was maintained at 70°C(oil phase). The oil phase was added to the water phase and pre-emulsification was carried out, followed by homogeneous emulsification by a homogenizer. After this, the system was rapidly cooled while being stirred to obtain the aforementioned emulsion.

### [Example 4] Foam pack

| (Ingredients) | Blended amount (wt%) |
|---|---|
| (1) Thiotaurine | 0.1 |
| (2) 1,3-butylene glycol | 5.0 |
| (3) Glycerine | 7.0 |
| (4) Methyl paraben | 0.1 |
| (5) Potassium hydroxide | 0.15 |
| (6) Stearic acid | 0.5 |
| (7) Myristic acid | 1.0 |
| (8) Batyl alcohol | 1.5 |
| (9) Polyoxyethylene (60) hardened castor oil | 3.0 |
| (10) Perfume | 0.05 |
| (11) Liquid petroleum gas | 6.0 |
| (12) Dimethyl ether | 3.0 |
| (13) Purified water | Balance |

### 〈Preparation method〉

(1)-(5) were added to (13) and dissolved by heating up to 70°C. (6)-(10), stirred and heated up to 75 °C , were added to this mixture, followed by thorough stirring and cooling. After filling a container with this mixture, (11) and (12), spraying agents, were put into the container to obtain the aforementioned foam pack.

### [Example 5] Ointment

| (Ingredients) | Blended amount (wt%) |
|---|---|
| (1) Thiotaurine | 0.1 |
| (2) Tocopherol acetate | 1.0 |
| (3) Stearyl alcohol | 18.0 |
| (4) Japanese core wax | 20.0 |
| (5) Polyoxyethylene (20) monooleate | 0.25 |
| (6) Glycerine monostearate | 0.3 |
| (7) Vaseline | 40.0 |
| (8) Purified water | Balance |

### 〈Preparation method〉

Thiotaurine was added to purified water and the temperature was maintained at 70°C (water phase). The rest of the ingredients were mixed and dissolved at 70°C (oil phase). The oil phase was added to the water phase and homogeneously emulsified with a homogenizer, followed by cooling to obtain the aforementioned ointment.

### [Example 6] Powdery foundation

| (Ingredients) | Blended amount (wt%) |
|---|---|
| | |

| Powder: | |
|---|---|
| Talc | 20.3 |
| Mica | 35.0 |
| Kaolin | 5.0 |
| Titanium dioxide | 10.0 |
| Mica titanium | 3.0 |
| Zinc stearate | 1.0 |
| Red iron oxide | 1.0 |
| Yellow iron oxide | 3.0 |
| Black iron oxide | 10.2 |
| Nylon powder | 10.0 |

| Binding agent: | |
|---|---|
| Squalane | 6.0 |
| Lanolin acetate | 1.0 |
| Octyldodecyl myristate | 2.0 |
| Diisooctanoic acid neopentyl alcohol | 2.0 |
| Sorbitan monooleate | 0.5 |

| Others: | |
|---|---|
| Thiotaurine | 0.1 |
| Preservative, antioxidant | Appropriate amount |
| Perfume | Appropriate amount |

### 〈Preparation method〉

The aforementioned powdery foundation was prepared using a conventional method.

### INDUSTRIAL APPLICABILITY OF THE INVENTION

As described thus far, the liniment composition for preventing environmental stress pertaining to the present invention is suitable for eliminating the stress which has adverse effects on the skin among types of stress due to floating fine particles which float in the air.

## Claims

1. A liniment composition for preventing environmental stress which comprises one or more types of ingredients selected from among a group consisting of sulfur containing amino acids, metabolic intermediates of sulfur containing amino acids, tannin and vitamin C and its derivatives.

2. The liniment composition for preventing environmental stress of claim 1 wherein the sulfur containing amino acid is glutathione and the metabolic intermediate of the sulfur containing amino acid is thiotaurine or hypotaurine.

3. The liniment composition for preventing environmental stress of claim 1 or claim 2 wherein hydroxycarboxylic acid or its derivative is additionally added to this composition.
